(19) Europäisches Patentamt
European Patent Office
Office européen des brevets

(11) **EP 2 081 014 A1**

(12) **EUROPEAN PATENT APPLICATION**

(43) Date of publication:
**22.07.2009 Bulletin 2009/30**

(51) Int Cl.:
*G01N 21/35* (2006.01)  *G01N 33/02* (2006.01)

(21) Application number: **09150769.9**

(22) Date of filing: **16.01.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **18.01.2008 JP 2008009554**

(71) Applicant: **Sumitomo Electric Industries, Ltd.**
**Osaka-shi, Osaka 541-0041 (JP)**

(72) Inventors:
• **Shimazu, Takayuki**
**c/o Yokohama Works of Sumitomo Electric Ind., Ltd**
**Yokohama-shi, Kanagawa (JP)**

• **Katayama, Makoto**
**c/o Yokohama Works of Sumitomo Electric Ind., Ltd**
**Yokohama-shi, Kanagawa (JP)**
• **Okuno, Toshiaki**
**c/o Yokohama Works of Sumitomo Electric Ind., Ltd**
**Yokohama-shi, Kanagawa (JP)**
• **Tanaka, Masato**
**c/o Yokohama Works of Sumitomo Electric Ind., Ltd**
**Yokohama-shi, Kanagawa (JP)**

(74) Representative: **Kreutzer, Ulrich et al**
**Cabinet Beau de Loménie**
**Bavariaring 26**
**80336 München (DE)**

(54) **Method of inspecting food and inspection apparatus implementing the same**

(57)    A method of inspecting food (50) which can evaluate quality of the food with improved precision includes steps of irradiating probing light (L1) having near infrared wavelengths on the food (50); detecting light (L3) diffusively reflected by the food (50) to obtain a reflectance spectrum; obtaining an absorbance spectrum based on the reflectance spectrum; and evaluating the food (50) using the reflectance spectrum and the absorbance spectrum. An apparatus for implementing the method includes: a light source unit (10) to generate probing light which has near infrared wavelengths and illuminates the food (50); a detector unit (20) to detect light diffusively reflected by the food (50) to obtain a reflectance spectrum of the food; and a processing unit (40) to evaluate the food based on the reflectance spectrum and an absorbance spectrum calculated from the reflectance spectrum.

**FIG. 1**

## Description

BACKGROUND OF THE INVENTION

Field of the Invention

The present invention relates to a method of inspecting food by irradiating probing light on the food and analyzing the diffuse reflection light caused by the probing light and an inspection apparatus implementing the method.

Description of the Background Arts

Inspection of food during processing thereof has been further important as consumers pay further attention to safety of the food.

Various techniques have been proposed and practically carried out to detect foreign materials or something unusual in the food.

For instance, a visual or X-rays inspection or an inspection using a metal detector or magnetic sensor has been used in the field of detecting whether processed food contains foreign materials or not. These techniques are often applicable only for a specific substance. For instance, an inspection using the metal sensor may detect a metal substance but it is ineffective for hair at all. An inspection using the X-rays, although the X-rays makes it possible to inspect the food from the outside of the package of the food, is ineffective also for hair because hair is transparent for the X-rays; or the irradiation of the X-rays on the food it self sometimes cause problems.

Another technique has been used to inspect quality of the food, in which content of moisture or sugar in the food is measured and the quality of the food is determined through the measured content. By drying a sample of the food under a reduced pressure, the moisture content therein may be measured. Because this measurement is a type of the destructive inspection, total inspection becomes impossible. Still another technique to evaluate moisture content measures the electrical conductivity of processed food. However, such a technique is necessary to contact a probe to the processed food to measure the conductivity, which leaves a subject in a hygiene viewpoint. Thus, known techniques are not always adequate for inspecting quality of processed food.

Japanese Patent Application published as JP 2004-301690A has proposed another technique that enables the total inspection and clears the hygiene subject. The technique irradiates light having visible and near infrared wavelengths on the processed food and analyzes the reflection from the food to detect a foreign material.

However, the processed food contains a plurality of materials including the base food thereof and a trace of foreign materials. Accordingly, the reflectance spectrum taken for the near infrared light shows blurred behaviors originated from the foreign materials, which degrades the accuracy of the detection of the foreign material. A superior technique to detect the foreign material in the processed food and to inspect the quality thereof has been requested.

SUMMARY OF THE INVENTION

The object of the present invention is to provide a method of inspecting food which can evaluate quality of the food with improved precision and to provide an apparatus for implementing the method.

The present invention provides a method of inspecting food, the method including steps of: irradiating probing light having near infrared wavelengths on the food; detecting light diffusively reflected by the food to obtain a reflectance spectrum; obtaining an absorbance spectrum based on the reflectance spectrum; and evaluating the food using the reflectance spectrum and the absorbance spectrum.

Also, the present invention provides an inspection apparatus to inspect food, the apparatus including: a light source unit to generate probing light which has near infrared wavelengths and illuminates the food; a detector unit to detect light diffusively reflected by the food to obtain a reflectance spectrum of the food; and a processing unit to evaluate the food based on the reflectance spectrum and an absorbance spectrum calculated from the reflectance spectrum.

The above-mentioned features and other features, aspects, and advantages of the present invention will be better understood through the following description, appended claims, and accompanying drawings. In the explanation of the drawings, an identical mark is applied to identical elements and an overlapping explanation will be omitted.

BRIEF DESCRIPTION OF THE DRAWINGS

Figure 1 is a schematic view showing an apparatus for inspecting food of the first embodiment of the present invention;

Figure 2 is a graph showing diffuse reflectance spectra measured by the apparatus shown in Fig. 1, wherein the doted line is a spectrum of light diffusively reflected by food and the solid line is a spectrum of light diffusively reflected by a foreign material;

Figure 3 is a graph showing KM absorbance spectra obtained from the diffuse reflectance spectra shown in Fig. 2, wherein the doted line is a spectrum regarding the food and the solid line is a spectrum regarding the foreign material;

Figure 4 is a graph showing the second differentiation spectra of the diffuse reflectance spectra shown in Fig. 2, wherein the doted line is a spectrum regarding the food and the solid line is a spectrum regarding the foreign material;

Figure 5 is a graph showing the second differentiation spectra of the KM absorbance spectra shown in Fig. 3, wherein the doted line is a spectrum regarding the food and the solid line is a spectrum regarding the foreign material; and

Figure 6 is a schematic view showing an apparatus for inspecting food of the second embodiment of the present invention.

DETAILED DESCRIPTION OF THE INVENTION

First Embodiment

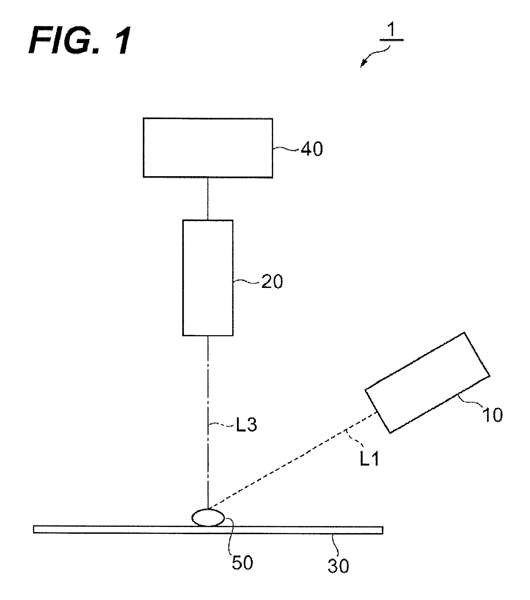

Figure 1 is a schematic view showing an apparatus for inspecting food (food tester 1) of the first embodiment of the present invention. The food tester 1 comprises a light source unit 10, a detector unit 20, a test stage 30, and a processing unit 40.

The light source unit 10 generates probing light having near infrared wavelengths and the probing light illuminates an area where an object 50 under inspection is placed. (The probing light has wavelengths from 800 to 2500 nm, preferably, from 1000 to 2500 nm.)

The light source 10 may be a halogen lamp, or may be a supercontinuum light source (SC light source). The SC light source includes a seed light source and a medium, whose optical characteristic appears a non-linearity with respect to the input optical power, and outputs SC light by injecting seed light therein and by widening the optical output spectrum therefrom due to the non-linearity of the optical characteristic of the medium. The SC light source may suppress the heating of the object 50 compared with the case of the halogen lamp; accordingly, the food tester 1 including the SC light source can inspect a food which must prevent heating. The light source 10 preferably has a function to modulate the probing light.

The detector unit 20 detects the light which is caused by the irradiation of the probing light from the light source 10 and is diffusively reflected at the surface of the object 50 as a diffuse reflectance spectrum. The information concerning to the diffuse reflectance spectrum is sent to the processing unit 40. The detector unit 20 has an optical detector whose primary material is made of, for instance, HgCdTe, InGaAs, and so on.

The test stage 30 on which the object is put may be made of material transparent to the probing light.

The processing unit 40 receives the information concerning to the diffuse reflectance spectrum from the detector unit 20 and analyzes the features thereof. The processing unit 40 derives the absorption spectrum, the second differentiation of the diffuse reflectance spectrum, and the second differentiation of the absorption spectrum. Moreover, some statistical calculations for those spectra are carried out in the processing unit 40.

The inspecting modes of the food tester 1 according to the present embodiment are to find foreign materials in the object 50 and to inspect the quality of the object 50. Here, explanation exemplarily concentrates on a case to find the foreign materials.

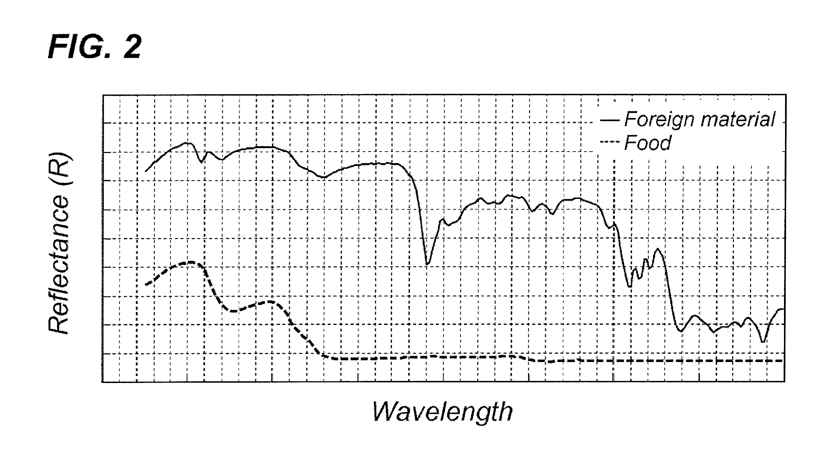

Figure 2 is a graph showing diffuse reflectance spectra measured, from 1000 to 2500 nm, by the food tester 1. In the Fig. 2, the doted line is a spectrum of light diffusively reflected by food, specifically raisins, and the solid line is a spectrum of light diffusively reflected by a foreign material, specifically a twig. The diffuse reflectivity of the food appears less distinguishable behavior and relatively small in a whole range of the wavelengths under inspected. While, the diffuse reflectance spectrum of light diffusively reflected by a foreign material shows some distinguishable features.

The absorbance spectrum (the KM absorbance) may be obtained by performing the Kubelka-Munk conversion (KM conversion) for the diffuse reflectance spectrum shown in Fig. 2. The KM conversion is given by the following equation:

$$\frac{K}{S} = \frac{(1-R)^2}{2R},$$

for the diffuse reflectance at respective wavelengths where K is the absorption co-efficient and S is the scattering co-efficient.

In wavelengths where the object shows larger absorption, only the diffusively reflected light through shorter path length can be radiated to detect because the light through longer path length is almost absorbed by the object. In wavelengths where the object shows less absorption, the diffusively reflected light through longer path length can be radiated from the object. Accordingly, relative magnitude of peaks in a diffuse reflectance spectrum, for example shown in Fig. 2, is weaker than relative magnitude of peaks in the corresponding absorption spectrum. The parameter K/S described above, namely the absorbance, may clarify the absorption behavior of the object and the inherent features thereof, which enhances the inspection quality for the object.

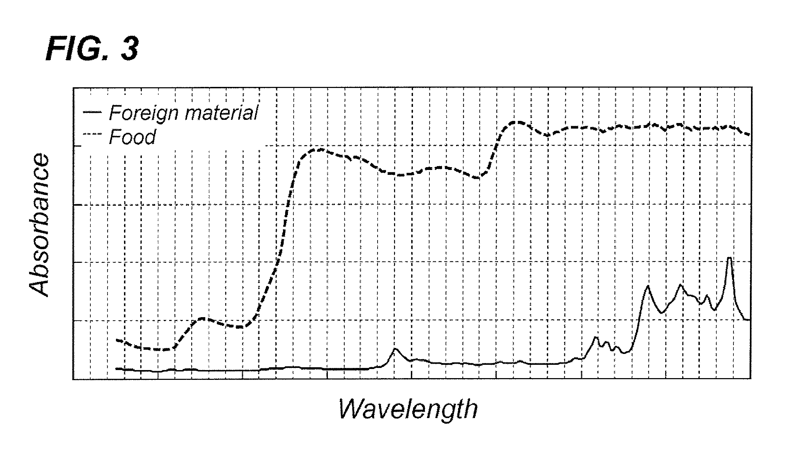

Figure 3 is a graph showing KM absorbance spectra obtained from the diffuse reflectance spectra shown in Fig. 2, wherein the doted line is a spectrum regarding the food and the solid line is a spectrum regarding the foreign material. The KM absorbance spectrum makes several peaks conspicuous, which are blurred in the diffuse reflectance spectrum of Fig. 2. Thus, the object can be evaluated with higher accuracy based on both of the diffuse reflectance spectrum and KM absorbance spectrum obtained from the diffuse reflectance spectrum.

The inspection for the food according to an embodiment of the present invention takes both the diffuse reflectance spectrum of Fig. 2 and the KM absorbance spectrum of Fig. 3 into account to evaluate the quality of the food under inspection. Specifically, the method first takes the diffuse reflectance spectrum of the food as a reference and obtains the reference KM absorbance spectrum through the reference reflectance spectrum in advance to the practical inspection. Second, the method takes the diffuse reflectance spectrum for the object, which might contain the foreign materials, and calculates the KM absorbance thereof. Then, the method compares the reflectance spectrum and the absorbance spectrum with those reference spectra, respectively, to determine whether the object contains the foreign material or not.

Moreover, the method can identify the foreign material in a base food by preparing the reference spectra of the foreign material. The foreign material to be contained within the base food is assumed to be, for instance, those originated from a human body, typically a hair, those originated from machines used in the food processing, typically metals, and contaminants within the base food. Pre-measurement of the diffuse reflectance spectrum and the KM absorbance spectrum for those materials makes the identification of the foreign material easily possible comparing the spectra with those practically measured for the object.

The selection of the diffuse reflectance spectrum or the KM absorbance spectrum may depend on the magnitude of the reflectance in the diffuse reflectance spectrum. As an example, when the reflectivity is greater than 0.1 (10 %), which corresponds to a case that the depth of the steep valley (the absorption peak) in the reflectance spectrum is greater than 0.1, the reflectance spectrum is preferable because the reflectance valley (the absorption peak) definitely identifies the wavelength thereof and the shape of the valley. While, in a case the reflectance is smaller than 0.1 (10 %), it is preferable to calculate the KM absorbance spectrum and to evaluate the foreign material after identifying respective peaks of the KM absorbance spectrum, because the reflectance spectrum occasionally blurs the behavior for the base food and that for the foreign material.

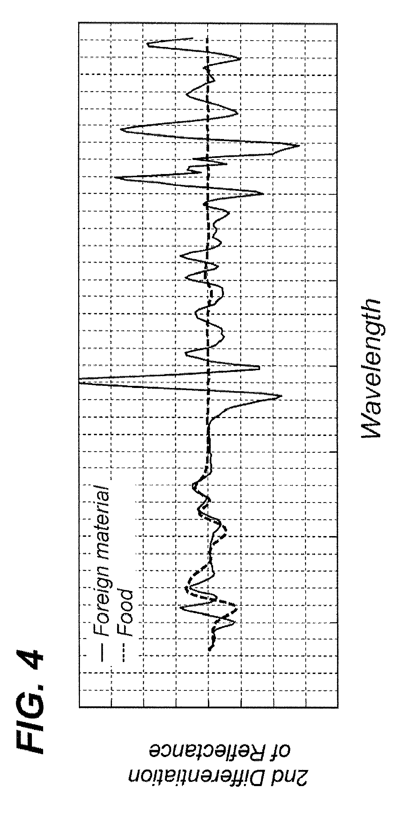

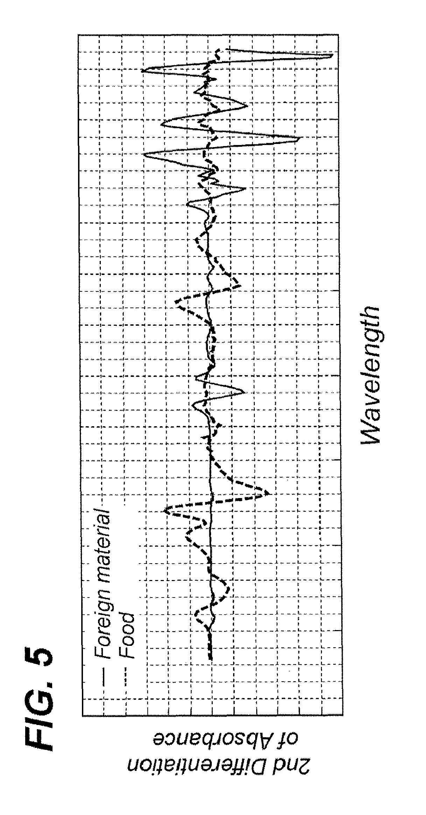

Figure 4 is a graph showing the second differentiation spectra of the diffuse reflectance spectra shown in Fig. 2. Figure 5 is a graph showing the second differentiation spectra of the KM absorbance spectra shown in Fig. 3. In Figs 4 and 5, the doted line is a spectrum regarding the food and the solid line is a spectrum regarding the foreign material. Thus, the second differentiation can make the behaviors conspicuous although the valley and the peak are reversed. Whether the object includes the foreign material or not can be determined in further preciseness by evaluating the peak wavelength and the magnitude thereof in the second differentiation of the reflectance spectrum and the KM absorbance spectrum.

The references of the second differential spectra of the reflectance and the absorbance are also preferable to evaluate the quality of the base food and to identify the origin of the foreign material. Concurrent with the measurement of the reference diffuse reflectance spectrum and its KM absorbance spectrum for the substance considered to be mixed within the base food, the second differentiation of those reference may be prepared as the reference of the second differentiation spectrum of those substance. Comparing the second differential spectrum of the object 50 with the references, the inspection of the object may not only determine whether the object 50 contains the foreign material but identify what substance is contained therein.

Also, it is efficient to compare the relative magnitude of the peaks (or valleys) appeared in the second differentiation of the reflectance spectrum or in the second differentiation of the KM absorbance spectrum for evaluating the quality of the base food. For instance, the relative magnitude of the peak at the wavelength of 1930 nm with respect to the peak at the wavelength of 1421 nm may sometimes determine whether the base food 50 contains the foreign material or not. By comparing the relative magnitude of the peaks, change of the magnitude of the peaks caused by fluctuation of the base line can be suppressed.

The calculation of the second differentiation of the reflectance spectrum and that of the KM absorbance spectrum preferably include substeps of: discretizing said reflectance spectrum and said absorbance spectrum with an interval greater than 30 nm; and calculating said second differentiation of said reflectance spectrum and said second differentiation of said absorbance spectrum with a numerical method. A lesser interval occasionally makes the noise inherently attributed to the inspection apparatus conspicuous and become comparable with the peak due to the foreign material. The interval greater than 30 nm may reduce the noise and makes it possible to distinguish the peaks of the object 50.

As described above, the KM absorbance spectrum calculated in the processing unit 40 in addition to the raw reflectance spectrum measured at the detector unit 20 can enhance the accuracy to detect whether the object 50 contains the foreign material or not.

Next will describe a method of inspecting the quality of the object 50 by the inspection apparatus 1 according to the present invention. One typical and well-known method of inspecting the quality of the food as the object 50 is to measure the moisture and to detect the sugar content within the food. The inspection apparatus 1 according to the present embodiment may measure the moisture and the sugar content in the object 50.

For instance, it is well known that the moisture in the food makes absorption peaks around 1400 nm and around 1900 nm. Accordingly, the magnitude of the absorption peaks around 1400 nm and that around 1900 nm may become an index of the moisture content in the food 50. Similarly, it is also well known that the sugar forms an absorption peak around 1500 nm and another absorption peak around 2100 nm; accordingly, the measurement of the peak wavelengths and the comparison of the absorbance of respective peaks may identify the types of the sugar and the content thereof. The method of inspecting the quality of the food makes it possible to produce the food with further enhanced quality by setting the threshold for the content of the moisture and the sugar and by discriminating the product showing out of range as an inferior product.

Thus, the inspection apparatus 1 according to the present embodiment may enhance the quality of the inspection by using the reflectance spectrum obtained by the irradiation of the neat infrared light on the object 50, the KM absorbance spectrum derived from the reflectance spectrum, and sometimes the second differentiation of those spectra.

Second embodiment

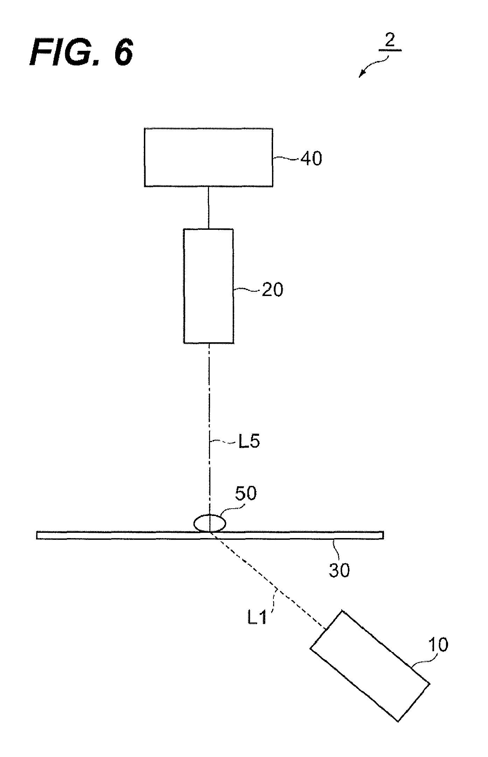

Figure 6 is a schematic view showing an apparatus for inspecting food (food tester 6) of the second embodiment of the present invention. The apparatus 2 is different from the first embodiment in a point that the light source 10 is located under the test stage 30 and the probing light L1 illuminates the object 50 through the stage 30.

The scattered light L5 is detected by the detector unit 20 and the inspection apparatus 2 obtains the diffuse reflectance spectrum in the processing unit 40 for the light detected by the detector unit 20. The processing unit 40 may also perform the calculation to obtain the KM absorbance spectrum, and the second differentiation of the reflectance and the absorbance spectra to inspect the quality of the object 50.

While this invention has been described in connection with what is presently considered to be the most practical and preferred embodiments, the invention is not limited to the disclosed embodiments, but on the contrary, is intended to cover various modifications and equivalent arrangements included within the spirit and scope of the appended claims. For instance, the wavelength of the probing light may be optional. The wavelength of the proving light may narrow in the range thereof to detect a specific foreign material and to measure the content in the food, or intermittently select a plurality of wavelength regions. When the wavelength region is narrowed, the inspection may be further precise to set the wavelength region of the probing light including at least 100 nm longer and at least 100 nm shorter than a wavelength where the foreign material shows the absorption peak.

The inspection apparatus may further perform the correction for the reflectance spectrum, the KM absorbance spectrum, and the second differentiation of both spectra. The correction of the spectra may be, for instance, the multiplicative scattering correction (MSC) for the base line to suppress the dependence on the surface roughness and so on appeared in the spectra. The present invention, therefore, is limited only as claimed below and the equivalents thereof.

The entire disclosure of Japanese Patent Application No. 2008-009554 filed on January 18, 2008 including specification, claims drawings, and summary are incorporated herein by reference in its entirety.

**Claims**

1. A method of inspecting food, the method comprising steps of:

   irradiating probing light having near infrared wavelengths on said food;
   detecting light diffusively reflected by said food to obtain a reflectance spectrum;
   obtaining an absorbance spectrum based on said reflectance spectrum; and
   evaluating said food by using said reflectance spectrum and said absorbance spectrum.

2. A method of inspecting food according to claim 1, further comprising a step of calculating a second differentiation of said reflectance spectrum and a second differentiation of said absorbance spectrum, wherein said evaluating step is performed by using said second differentiation of said reflectance spectrum and said second differentiation of said absorbance spectrum in addition to said reflectance spectrum and said absorbance spectrum.

3. A method of inspecting food according to claim 2, wherein said calculating step includes substeps of: discretizing said reflectance spectrum and said absorbance spectrum with an interval greater than 30 nm; and calculating said second differentiation of said reflectance spectrum and said second differentiation of said absorbance spectrum with a numerical method.

4. A method of inspecting food according to claim 2, wherein said evaluating step includes a substep of comparing magnitude of a plurality of peaks appeared in said second differentiation of said reflectance spectrum and in said second differentiation of said absorbance spectrum.

5. A method of inspecting food according to claim 1, further comprising a step of obtaining a reference reflectance spectrum by irradiating said probing light on an object made of substantially same material with said food under inspection,
   wherein said evaluating step includes substeps of comparing said reflectance spectrum with said reference reflectance spectrum; and determining whether said food contains a foreign material based on said comparing substep.

6. A method of inspecting food according to claim 1, further comprising steps of: obtaining a reference reflectance spectrum by irradiating said probing light on an object made of substantially same material with said food under inspection; and obtaining a reference absorbance spectrum based on said reference reflectance spectrum,

wherein said evaluating step includes sub steps of: comparing said absorbance spectrum with said reference absorbance spectrum; and determining whether said food contains a foreign material based on said comparing step.

7. A method of inspecting food according to claim 1, wherein said evaluating step includes a substep of evaluating moisture content in said food based on behaviors of said reflectance spectrum and said absorbance spectrum.

8. A method of inspecting food according to claim 1, wherein said evaluating step includes a substep of evaluating sugar content in said food based on behaviors of said reflectance spectrum and said absorbance spectrum.

9. An inspection apparatus to inspect food, said apparatus comprising:

   a light source unit to generate probing light which has near infrared wavelengths and illuminates said food;
   a detector unit to detect light diffusively reflected by said food to obtain a reflectance spectrum of said food; and
   a processing unit to evaluate said food based on said reflectance spectrum and an absorbance spectrum calculated from said reflectance spectrum.

10. An inspection apparatus according to claim 9, wherein said processing unit calculates a second differentiation of said reflectance spectrum and a second differentiation of said absorbance spectrum, and said processing unit evaluates said food based on said second differentiation of said reflectance spectrum and said second differentiation of said absorbance spectrum in addition to said reflectance spectrum and said absorbance spectrum.

11. An inspection apparatus according to claim 10, wherein said processing unit discretizes said reflectance spectrum and said absorbance spectrum with an interval greater than 30 nm and calculates said second differentiation of said reflectance spectrum and said second differentiation of said absorbance spectrum with a numerical method.

12. An inspection apparatus according to claim 11, wherein said processing unit compares magnitude of a plurality of peaks appeared in said second differentiation of said reflectance spectrum or in said second differentiation of said absorbance spectrum.

13. An inspection apparatus according to claim 10, wherein, to determine whether said food contains a foreign material, said processing unit compares said reflectance spectrum with a reference reflectance spectrum which is obtained in advance to said inspection of said food by irradiating said probing light on an object made of substantially same material with said food under inspection.

14. An inspection apparatus according to claim 10, wherein, to determine whether said food contains a foreign material, said processing unit compares said absorbance spectrum with a reference absorbance spectrum which is obtained in advance to said inspection of said food by irradiating said probing light on an object made of substantially same material with said food under inspection,.

15. An inspection apparatus according to claim 10, wherein said processing unit evaluates moisture content in said food based on behaviors of said reflectance spectrum and said absorbance spectrum.

16. An inspection apparatus according to claim 10, wherein said processing unit evaluates sugar content in said food based on behaviors of said reflectance spectrum and said absorbance spectrum.

# FIG. 1

## FIG. 2

Legend: — Foreign material / ---- Food

Y-axis: Reflectance (R)

X-axis: Wavelength

EP 2 081 014 A1

FIG. 3

EP 2 081 014 A1

## FIG. 4

2nd Differentiation
of Reflectance

— Foreign material
---- Food

Wavelength

# FIG. 5

EP 2 081 014 A1

**FIG. 6**

Europäisches
Patentamt
European
Patent Office
Office européen
des brevets

**EUROPEAN SEARCH REPORT**

Application Number

EP 09 15 0769

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 324 945 A (IWAMOTO MUTSUO [JP] ET AL) 28 June 1994 (1994-06-28) * column 1, line 55 - line 64 * * column 2, line 62 - column 4, line 11; claim 1 * ----- | 1-16 | INV. G01N21/35 G01N33/02 |
| X | WALSH K B ET AL: "Sorting of fruit using near infrared spectroscopy: application to a range of fruit and vegetables for soluble solids and dry matter content" J. NEAR INFRARED SPECTROSCOPY, [Online] vol. 12, no. 3, 2004, pages 141-148, XP002524259 Retrieved from the Internet: URL:http://www.impublications.com/subs/jnirs/v12/J12_0141.pdf> [retrieved on 2009-04-20] * page 142, column 2, line 5 - page 144, column 1, line 12 * * page 144, column 2, line 8 - page 145, column 1, line 3; figure 2 * ----- | 1-16 | |
| A | WO 2007/083533 A (SUMITOMO ELECTRIC INDUSTRIES [JP]; OKUNO TOSHIAKI [JP]) 26 July 2007 (2007-07-26) | 1-16 | TECHNICAL FIELDS SEARCHED (IPC) G01N |
| P,A | -& EP 1 923 692 A (SUMITOMO ELECTRIC INDUSTRIES [JP]) 21 May 2008 (2008-05-21) * paragraph [0042] - paragraph [0048]; figure 3 * ----- | | |
| A | US 2004/012781 A1 (GEHRLEIN LANE [US] ET AL) 22 January 2004 (2004-01-22) * claims 45,46,49,50,54-59 * ----- -/-- | 1-16 | |

The present search report has been drawn up for all claims

| Place of search | Date of completion of the search | Examiner |
|---|---|---|
| Munich | 20 April 2009 | Consalvo, Daniela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 09 15 0769

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | PICTIAW C: "USE OF OPTICAL PROPERTIES OF FOOD MATERIALS IN QUALITY EVALUATION AND MATERIALS SORTING" JOURNAL OF FOOD PROCESSING ENGINEERING, [Online] vol. 2, 1978, pages 307-322, XP002524289 Retrieved from the Internet: URL:http://www3.interscience.wiley.com/cgi-bin/fulltext/119617907/PDFSTART> [retrieved on 2009-04-20] * figures 5,6; table 1 * ----- | 1-16 | |
| | | | TECHNICAL FIELDS SEARCHED (IPC) |

The present search report has been drawn up for all claims

| Place of search | Date of completion of the search | Examiner |
|---|---|---|
| Munich | 20 April 2009 | Consalvo, Daniela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 15 0769

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2009

| Patent document cited in search report | | Publication date | Patent family member(s) | | Publication date |
|---|---|---|---|---|---|
| US 5324945 | A | 28-06-1994 | JP | 2517858 B2 | 24-07-1996 |
| | | | JP | 6186159 A | 08-07-1994 |
| WO 2007083533 | A | 26-07-2007 | CN | 101375152 A | 25-02-2009 |
| | | | EP | 1923692 A1 | 21-05-2008 |
| | | | JP | 2007192750 A | 02-08-2007 |
| EP 1923692 | A | 21-05-2008 | CN | 101375152 A | 25-02-2009 |
| | | | JP | 2007192750 A | 02-08-2007 |
| | | | WO | 2007083533 A1 | 26-07-2007 |
| US 2004012781 | A1 | 22-01-2004 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004301690 A

- JP 2008009554 A